Europäisches Patentamt

European Patent Office

Office européen des brevets

(19)

(11) Publication number: **0 144 384**
**B1**

(12) **EUROPEAN PATENT SPECIFICATION**

(45) Date of publication of patent specification: **21.09.88**

(21) Application number: **84902164.7**

(22) Date of filing: **11.05.84**

(86) International application number:
**PCT/US84/00710**

(87) International publication number:
**WO 84/04657 06.12.84 Gazette 84/28**

(51) Int. Cl.⁴: **A 23 K 1/00,** A 61 K 9/32

(54) **RUMEN-STABLE PELLETS.**

(30) Priority: **26.05.83 US 498445**

(43) Date of publication of application:
**19.06.85 Bulletin 85/25**

(45) Publication of the grant of the patent:
**21.09.88 Bulletin 88/38**

(84) Designated Contracting States:
**CH DE FR GB LI NL SE**

(56) References cited:
**WO-A-84/00282**
**US-A-4 181 708**
**US-A-4 181 709**

(73) Proprietor: **EASTMAN KODAK COMPANY**
**343 State Street**
**Rochester New York 14650 (US)**

(72) Inventor: **WU, Stephen, Hong-Wei**
**1104 Meadow Lane**
**Kingsport, TN 37663 (US)**

(74) Representative: **Parent, Yves et al**
**Kodak-Pathé Département Brevets et Licences**
**Centre de Recherches et de Technologie Zone Industrielle**
**F-71102 Chalon-sur-Saône Cédex (FR)**

Courier Press, Leamington Spa, England.

## Description

Technical field

This invention relates in general to pellets adapted to be orally administered to ruminants and which are beneficial to ruminants after passing the rumen and reaching the abomasum and/or intestines. More particularly, this invention relates to pellets having, in terms of structure, a core material such as a nutrient or medicament and a coating over the core material which protects the core in the environment of the rumen, but which loses continuity under the more acidic conditions of the abomasum to render the core material available for utilization by the animal.

Background of the art

In ruminants, e.g., beef and dairy cattle, sheep, etc., ingested feed first passes into the rumen, where it is pre-digested by fermentation. During this period of fermentation the ingested feed may be regurgitated to the mouth where it is salivated and ruminated. After a period of fermentation, adsorption of digested nutrients starts and continues in the subsequent sections of the digestive tract. This digestive process is described in detail by D. C. Church, "Digestive Physiology and Nutrition of Ruminants", Vol. 1, O.S.U. Book Stores, Inc. of Corvallis, Oregon.

The rumen serves as an important location of metabolic breakdown of ingested foodstuffs through the action of microorganisms which are present therein. Ingested food is typically retained in the rumen for from about 6 to about 30 hours, during which time it is subject to metabolic breakdown by the rumen microorganisms. When the rumen contents pass into the abomasum and intestine, the microbial mass is digested, thus providing protein to the ruminant. Thus, the natural nutritional balance of the ruminant animal is primarily a function of the microbial composition and population.

In preparing nutrients and medicaments intended for administration to ruminants, it is important to protect the active ingredients against the environmental conditions of the rumen, i.e., microbial degradation and the effects of a pH of about 5.5, so the active substance will be saved until it reaches the particular location where adsorption takes place.

U.S. Patent 4,181,708 discloses rumen-stable pellets coated with a mixture of a polymeric material, a hydrophobic material, and a flake material. All these ingredients are as described herein for the present invention the flake material constituting from about 10 to 200% of the weight of the polymeric material. However, while the coating compositions of U.S. Patent 4,181,708 provide excellent protection of the core material, the problem has been to provide still higher levels of protection.

Disclosure of the invention

Improved protection is provided in the compositions of U.S. Patent 4,181,708 by providing specific amounts of the components of the coating composition. The present invention provides rumen stable pellets comprising a core material and a coating surrounding the core material, the coating comprising

a) copoly (2 - methyl - 5 - vinylpyridine/styrene) 80:20
b) stearic acid
c) talc/aluminum (1:1 w/w)

characterized in that said coating comprises 20—25% of (a), 5—15% of (b), 60—75% of (c), percentages being by weight on the basis of the total coating composition weight.

The polymeric material in combination with the hydrophobic subtance is physiologically acceptable and resistant to a pH of greater than about 5 but capable of releasing the core of the pellets at a pH of less than about 3.5. The coating makes up about 5 to about 50% of the weight of the pellet, and has a sticking temperature of at least about 50°C.

Brief description of drawing

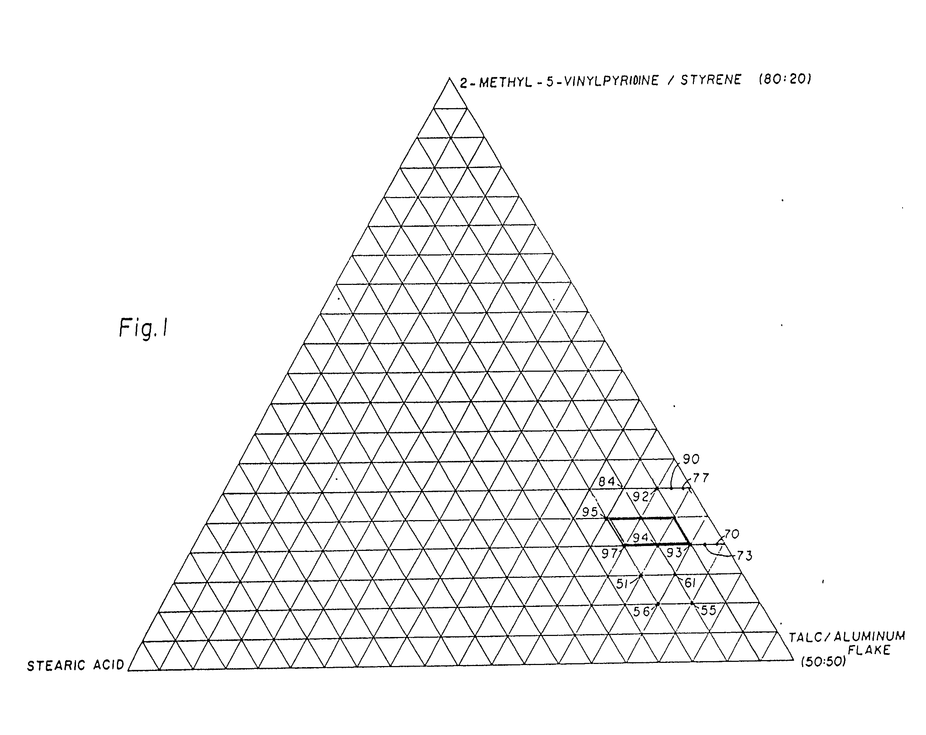

Figure 1 is a triangular coordinate graph illustrating the invention.

Modes for carrying out the invention

The pellets according to this invention are adapted for oral administration to ruminants. The pellets are of a suitable shape and size, such as cylindrical, having a diameter of about 1.0—2.0 mm and a length-to-diameter ratio of about 1—2.0:1. Also, the pellets must be of suitable density, i.e., a specific gravity of between about 1 and 1.4, have acceptable odor, taste, feel, etc. The pellets include a core and a continuous film or coating completely encapsulating the core.

The core is of a material beneficial to the ruminant upon passing the rumen and reaching the abomasum and/or intestine. Normally, the core is a solid material which has been formed into particles, such as by pelletizing prior to being coated. The cores should have sufficient body or consistency to remain intact during handling, particularly during the coating operation. Suitable core materials include various medicaments and nutrients such as, for example, antibiotics, relaxants, drugs, antiparasites, amino acids, proteins, sugars, carbohydrates, etc. The core may also contain inert filler material such as clay and acid or base neutralizers.

The core material may be made ready for coating by the following method. The nutrient, medicament, or the like, and core neutralizer, if used, are mixed with water, binders, and sometimes inert organic substances added to adjust the specific gravity of the pellet and the resulting plastic dough-like mass is extruded or rolled to obtain suitable size particles. Adhesive binders may be added to strengthen the pellets and can be nontoxic vegetable gums, starches, cellulose derivatives, animal gums and other similar substances well-known in the art of food thickening and tablet making. Inorganic additives used to adjust the specific gravity of the pellet include such substances as insoluble, nontoxic pigment-like materials such as metal sulfates, oxides and carbonates having a relatively high density. After creating suitable size pellets, the pellets are dried to remove the water. The pellets are then coated by contacting them with a solution of the protective coating material in a suitable solvent or mixture of solvents as hereinafter described.

The coating material is capable of forming a continuous film around the core by the evaporation of solvent from the coating material. It has the ability to withstand environmental conditions of the rumen, and the ability to expose the core material of the pellet in the environment of the abomasum. Thus, the coating material should be resistant to pH conditions of greater than about 5 for from about 6 to about 30 hours. The coating should release the core material after exposure to abomasum environmental conditions having a pH of about 2 to about 3.3. Release should occur within the residence time in the abomasum or later in the intestinal track but at least within a time period of six hours after contacting pH 3.5 or less. The exposure of the core may occur by the coating becoming permeable to the contents of the rumen, such as by dissolving, disintegrating, or extensive swelling. The coating material is physiologically acceptable, i.e., the coating material should not interfere with the ruminants' healthy or normal body functioning.

Another requirement for the coating material is its ability to withstand abrasion in handling and storage conditions of relatively high heat and/or humidity without a significant amount of blocking or sticking. It should have a sticking temperature of greater than about 50°C. Sticking temperature is defined as the temperature at which an applied force of 2.45 N/cm² (0.25 Kg/cm²) for 24 hours causes the coating of pellets to adhere to the coating of adjacent pellets strongly enough to cause rupture of the coating when the pellets are forceably separated. Also, the coating material is preferably soluble or dispersable in organic solvents having boiling points of between about 40°C. and 140°C. to permit conventional coating processes such as spray coating to be used. Particularly suitable solvents include methylene chloride, chloroform, ethanol, methanol, ethyl acetate, acetone, toluene, isopropanol or mixtures of these.

The polymeric substances for the coating include polymers, copolymers and mixtures of polymers and/or copolymers having basic amino groups in which the nitrogen content of the polymeric substance is between about 2 and about 14% of a film-forming molecular weight. Especially preferred are poly(vinylpyridine), polymeric derivatives of vinylpyridine, and the copolymers of the various isomers and derivatives of vinylpyridine copolymerized with one or more of the above-mentioned addition type monomers.

Included among the especially preferred copolymers are 2 - methyl - 5 - vinylpyridine and styrene, and in particular, the copolymer of about 80% by weight 2 - methyl - 5 - vinylpyridine and about 20% by weight styrene. These copolymers are commercially available or may be produced by conventional techniques well known in the art.

Hydrophobic substances which are physiologically acceptable and have the correct degree of compatibility with the polymer are commercially available. It is important that the polymer and hydrophobic substance have a degree of compatibility to permit the film to remain intact in the rumen environment, but to permit permeation of the abomasal fluid to the core while the pellet is in the abomasum.

A class of hydrophobic substances of value are fatty acids containing from 10 to 32 carbon atoms such as lauric, oleic, stearic, palmitic and linoleic. These substances are well known to be water insoluble due to the long hydrocarbon radical but to react to water due to the polar nature of the carboxyl group. In the selected basic amino group containing polymers, the carboxyl group of the fatty acid is able to react with the basic nitrogen group to form a weak salt-type linkage. This attachment to the polymer serves to cause the fatty acid to be fixed in the polymer matrix. The hydrophobic hydrocarbon chain of the fatty acid tends to render the matrix water resistant and thereby decreases swelling of the otherwise water susceptible polar film. Both the interior of the matrix film and the surface is now water resistant in aqueous environments at a pH above about 5.0. However, at pH values below pH 4.5 and especially below about pH 3.5 the affinity of the basic nitrogen group for water and the hydrogen ion overcomes the increased water resistance. The film reacts with the acid environment and loses barrier properties sufficient to allow the core material to escape to the environment.

It is believed that the function of the hydrophobic substance as a dispersed phase in the protective polymer layer is as follows:

a. reduces wetting of the coating and therefore initial attack by water,

b. reduces total volume of coating affected by water, and

c. extends the length of permeable pathway the water must travel to core.

In accordance with this invention, a physiologically acceptable flake material is dispersed throughout the polymeric matrix. The flake material is substantially inert with respect to the environment of the rumen.

Suitable inert flake materials include metal flake, mineral flake, crosslinked organic polymer, etc. Especially suitable are aluminum flake, talc, graphite, and ground mica. Aluminum flake is produced by ball-milling the aluminum in a liquid medium in the presence of a lubricant such as stearic acid. It is available commercially from Alcan Metal Powders, Division of Alcan Aluminum Corporation. Sizes of the flake are generally less than about 100 micrometers, preferably about 1—60 micrometers. Talc particle sizes are generally within the range of 0.5—40 micrometers. It is sometimes beneficial to subject the hydrophobic substance and flake material to ball-milling operations such that they are brought into rubbing contact for several hours, thereby increasing the efficiency of the coating.

In the practice of this invention, the polymeric material may conveniently be dissolved in a suitable solvent which would be physiologically acceptable in the event there are residues upon evaporation of the solvent, as hereinbefore described. The hydrophobic substance and flake material are blended in the solution, wherein the polymeric substance is a continuous matrix and the additives are dispersed therein. The coating solution may be applied by various well known means such as, for example, brushing, dipping, spraying, fluidized bed, etc.

The coating compositions disclosed in this invention are also useful for other bioactive materials, particularly highly water-soluble amino acids such as lysine, carbohydrates, vitamins, antibiotics, hormones, and other pharmaceuticals. In general, a highly water-soluble active ingredient is much more difficult to protect less water-soluble bioactive compound and a successful rumen-stable coating for highly water-soluble materials is obviously useful for materials of low water solubility so that the coating compositions in this invention may be viewed as an universal coating which is independent of the solubility of the core material.

Unless otherwise specified, all parts, percentages, ratios, etc., are by weight. Molecular weights are given as weight averages.

Examples

The examples which follow are submitted for a better understanding of the invention. While the examples are based on in vitro tests, the in vitro experiments shown in the examples simulate conditions existing in ruminants thereby permitting the study of coated pellets without the use of live animals. It has been determined by actual in vivo tests that the testing of pellets in the aqueous media used in the examples simulating the environmental conditions of the rumen and abomasum with respect to temperature, pH, etc., provide reliable data concerning the protection offered by the coatings in the rumen, and releasability of the coatings in the abomasum. Nutrients such as amino acids and proteins which may be used in the core material are known to be beneficial to ruminants when positioned in the intestinal tract downstream from the rumen.

Generally, pellets are prepared from the nutrients indicated to a size of between about 8 and 12 sieve size (2.38 mm—1.68 mm). The nutrients are mixed with conventional additives such as micro-crystalline cellulose, binders, inert consistency adjusting substances such as water. The pellets are formed by a conventional pelletizer, dried, sieved, and coated using a coater as described herein. Upon application of the coating to the pellets, they are tested for resistance to pH conditions resembling those of the rumen and abomasum by agitating in buffer solutions of pH 2.9 for 0.5 hours and 5.4 for 24 hours. Recovery and protection figures cited for active core ingredients herein contain in them all materials of the original coated pellet that are not completely dissolved in the pH 2.9 buffer, including any undissolved active ingredient in the original core. For the sake of simplicity abbreviations are used in the examples as follows:

2M5VP-2-methyl-5-vinylpyridine

ST-styrene

When coating ratios are used, the first number indicates the number of parts polymer, the second number indicates the number of parts hydrophobic substance, and the third number indicates the number of parts inert flake material.

Cylindrical smooth-edge glucose cores (−8/+12 mesh, U.S. standard mesh size (2.38 mm—1.68 mm) which consist of 90% glucose and 10% microcrystalline cellulose are used in all experiments. In the process of preparing glucose cores, glucose powder and microcrystalline cellulose are mixed thoroughly in a mixer-extruder; water is then added slowly to wet the dry solid mix until an extrudable dope was formed. The moisture content of the final dope is about 18%. The dope is extruded from the mixer-extruder through a die with 2-mm diameter holes and chopped with a rotating knife into small cylinders with a length/diameter ratio approximately equal to one. Wet cores are tumbled to smooth the rough edges, and then dried in an oven at 60°C. Dry cores are screened to yield uniform size cores for all coating experiments.

In preparing the coating dopes, a mixture of copoly (2M5VP/ST 80:20) (I.V.=1.30), 20—25% by weight; stearic acid, 5—15% by weight, and talc/aluminum flake (1:1 w/w), 60—75% by weight are mixed in acetone to make a series of coating dopes containing 5% solids. The exact coating compositions for the examples are shown as dots in the tricompositional diagram in Figure 1 (Comparative examples are also shown). The coordinates represent percentage of total composition weight of the polymeric material, hydrophobic substance and flake material. Glucose cores are coated with the coating dopes to desired coating levels using an air-suspension coater under the same operating conditions.

Rumen-protection and abomasal release are measured by extracting coated pellets in pH 5.4 buffer for 24 hours and pH 2.9 buffer for one hour respectively. Protection values are obtained for pellets with 6, 12, and 18% coating levels for each coating composition. The protection value at 15% coating weight for each coating composition was determined and indicated under the respective coating composition in Figure 1. For example, the rumen protection value of pellets coated with 20/70/10 (2M5VP/ST)/(Talc/Al)/stearic acid is 94%.

Unless otherwise specified, the inherent viscosity (I.V.) of the polymers is determined at 25°C., using 0.25 gram polymer per 100 ml of a solvent composed of dimethylformamide.

## Claims

1. Rumen stable pellets comprising a core material and a coating surrounding the core material said coating comprising

a) copoly (2 - methyl - 5 - vinylpyridine/styrene) 80:20

b) stearic acid

c) talc/aluminum (1:1 w/w)

characterized in that said coating comprises 20—25% of (a), 5—15% of (b), 60—75% of (c), percentages being by weight on the basis of the total coating composition weight.

2. A pellet according to claim 1 wherein (a) is 20—25%, (b) is 15%, (c) is 60—65%, percentages being by weight on the basis of the total coating composition weight.

## Patentansprüche

1. Pansen-stabile Pellets mit einem Kernmaterial und einer das Kernmaterial umhüllenden Beschichtung mit

(a) Copoly(2 - methyl - 5 - vinylpyridin/styrol) 80:20,

(b) Stearinsäure und

(c) Talcum/Aluminium (Gew.-Verhältnis 1:1), dadurch gekennzeichnet, daß die Beschichtung zusammengesetzt ist aus: 20 bis 25 Gew.-% aus (a), 5 bis 15 Gew.-% aus (b) und 60—75 Gew.-% aus (c), bezogen auf das Gesamtgewicht der Beschichtungszusammensetzung.

2. Pellet nach Anspruch 1, in dem in der Beschichtung (a) zu 20 bis 25 Gew.-%, (b) zu 15 Gew.-% und (c) zu 60 bis 65 Gew.-%, bezogen auf das Gesamtgewicht der Beschichtungszusammensetzung vorliegen.

## Revendications

1. Comprimés enrobés insensibles à l'action du rumen, comprenant une substance interne recouverte d'une couche composée

a) d'un copolymère 80:20 de méthyl - 2 - vinyl - 5 - pyridine et de styrène,

b) d'acide stéarique,

c) d'un mélange 1:1 en poids de talc et d'aluminium,

caractérisés en ce que ladite couche comprend 20 à 25% de (a), 5 à 15% de (b), 60 à 75% de (c), ces pourcentages étant exprimés en poids par rapport au poids total de la composition de la couche.

2. Comprimé enrobé selon la revendication 1 dans laquelle (a) est compris entre 20 et 25%, (b) est 15% et (c) est compris entre 60 et 65%, les pourcentages étant exprimés en poids par rapport au poids total de la composition de la couche.

Fig.1

2-METHYL-5-VINYLPYRIDINE / STYRENE (80:20)

STEARIC ACID

TALC/ALUMINUM
FLAKE
(50:50)

0 144 384